# EUROPÄISCHE PATENTSCHRIFT

(11) **EP 2 579 911 B1**
(45) Veröffentlichungstag und Bekanntmachung des Hinweises auf die Patenterteilung: **30.04.2014**
(21) Anmeldenummer: 11724635.5
(22) Anmeldetag: 10.06.2011
(51) Int. Cl.: A61M 1/14, A61M 1/16, G01N 21/64

(54) **DIALYSEANORDNUNG MIT EINER NACHWEISVORRICHTUNG**
DIALYSIS ASSEMBLY WITH A DETECTION DEVICE
ENSEMBLE DE DIALYSE AVEC DISPOSITIF DE DÉTECTION

(30) Priorität: 11.06.2010 DE 102010023486
(43) Veröffentlichungstag der Anmeldung: 17.04.2013
(73) Patentinhaber: B. Braun Avitum AG, 34212 Melsungen (DE)
(72) Erfinder: FRORIP, Aleksandr, EE-61508 Elva (EE); NACKE, Christoph, EE-51007 Tartu (EE)
(74) Vertreter: Arth, Hans-Lothar
(86) Internationale Anmeldenummer: PCT/EP2011/059651
(87) Internationale Veröffentlichungsnummer: WO 2011/154513

(56) Entgegenhaltungen:
- WO-A1-99/62574
- US-A1- 2009 293 646
- US-B2- 7 142 303

## Beschreibung

Die Erfindung betrifft eine Dialyseanordnung mit einer Nachweisvorrichtung zum quantitativen Nachweis vorbestimmter Moleküle, insbesondere von mittelgroßen Protein-Molekülen, in einer Probenflüssigkeit, mit einer Lichtquelle zur Aussendung von Messstrahlung, einem Probenflüssigkeits-Bestrahlungsabschnitt und einem Lichtdetektor zur Erfassung von den Probenflüssigkeits-Bestrahlungsabschnitt verlassender Nachweisstrahlung sowie einer dem Lichtdetektor nachgeordneten Auswertungsstufe zur Auswertung des Detektorsignals.

Mit Dialysebehandlungen wird der Körper eines Menschen (oder Tieres) mit eingeschränkter oder verlorengegangener Nierenfunktion bei der Entfernung von Abbauprodukten des Stoffwechsels aus dem Blut unterstützt, bis hin zur vollständigen Übernahme der Nierenfunktion durch die Dialyseanordnung. Bereits vor Jahren wurde es als wünschenswert erkannt, eine Dialysebehandlung in Abhängigkeit von ihrem Erfolg - also dem zeitabhängig erreichten Stand der Blutreinigung - zu steuern, um einerseits einen ausreichenden Effekt dieser umständlichen und für den Patienten beschwerlichen Behandlung zu gewährleisten und andererseits die Aufwendung unnötiger Behandlungszeit zu vermeiden. Es wurden Modellansätze zu r Beurteilung des Behandlungserfolges während der Behandlung entwickelt und Messgrößen gefunden, deren aktueller Wert hinreichend repräsentativ für den Behandlungserfolg ist und die während einer Behandlung erfasst werd en können.

Unter anderem kann der Harnstoffgehalt in der aus der Dialyseanordnung abgeführten, verbrauchten Dialyseflüssigkeit gemessen und als Maß für den Behandlungserfolg benutzt werden. Dies erfordert aber eine Probenahme und labortechnische Auswertung und ist daher kaum "online" während des Behandlungsvorganges einsetzbar. Daneben wurde ein Überwachungsverfahren unter Einsatz von Leitfähigkeits-Sensoren entwickelt, die enzymatisch induzierte Leitfähigkeitsänderungen der abgeführten Dialyseflüssigkeit erfassen, welche durch die Hydrolyse von Harnstoff (oder anderen maßgeblichen Molekülen) in der Dialyseflüssigkeit bewirkt werden. Bei diesem Verfahren sind allerdings praktische Probleme bei der Kalibrierung der Sensoren und der Kompensation einer (fallweise differierenden) Basis-Leitfähigkeit aufgetreten.

In der WO 99/62574 wird daher ein Verfahren zur Bestimmung des Gehaltes an Abbauprodukten in der Dialyseflüssigkeit während einer Dialysebehandlung vorgeschlagen, welches sich der spektralphotometrischen Analyse der Dialyseflüssigkeit bedient. In der WO 2008/000433 A1 werden ebenfalls ein spektroskopischer Detektor und ein Verfahren mit ähnlicher Anwendung vorgeschlagen, die speziell zum Nachweis von Blut und biologischen Markersubstanzen in Flüssigkeiten - etwa auch in Dialyseflüssigkeit - vorgesehen sind. Ein entsprechender Blutdetektor dient in Dialyseanordnung en insbesondere zur schnellen Erfassung von patientenkritischen Zuständen in Folge von Betriebsstörungen der Anordnung (etwa durch eine Membranruptur des Membranfilters, das Vertauschen von Anschlüssen oder eine Hämolyse), Die letztgenannte Druckschrift enthält auch eine Reihe von Quellenangaben für weiteren Stand der Technik.

Die US 2009/023646 offenbart eine Nachweisvorrichtung zum quantitativen Nachweis vorbestimmter Moleküle in einer Probenflüssigkeit, mit einer Lichtquelle zur Aussendung von Messstrahlung, einem Probenflüssigkeitsbestrahlungsabschnitt und einem Lichtdetektor zur Erfassung von den Probenflüssigkeitsbestrahlungsabschnitt verlassender Nachweisstrahlung sowie einer dem Lichtdetektor nachgeordneten Auswertungsstufe zur Auswertung des Detektorsignals, wobei die Lichtquelle derart ausgeführt ist, dass die Messstrahlung einen die Eigenfluoreszenz eines zu bestimmenden Moleküls anregenden Spektralbereich aufweist, und der Lichtdetektor derart ausgeführt ist, dass mindestens ein Spektralbereich der Nachweisstrahlung erfasst wird, in dem das Molekül Eigenfluoreszenzstrahlung emittiert.

Es ist Aufgabe der Erfindung, eine verbesserte Nachweisvorrichtung der erwähnten Art bereit zu stellen, welche hochgradig spezifisch und zuverlässig arbeiten und zur Überwachung und gegebenenfalls Steuerung von Dialysebehandlungen vorteilhaft einsetzbar sind.

Diese Aufgabe wird in ihrem Vorrichtungsaspekt durch eine Dialyseanordnung mit einer Nachweisvorrichtung mit den Merkmalen des Anspruchs 1 gelost. Zweckmäßige Ausgestaltungen der Erfindung sind Gegenstand der abhängigen Ansprüche.

Die Erfindung geht davon aus, die Eigenfluoreszenzstrahlung bestimmter Moleküle, die in einer zu untersuchend en Flüssigkeit regelmäßig enthalten sind und deren Gehalt zu bestimmen ist, für den quantitativen Nachweis zu nutzen. Sie schließt weiter den

Gedanken ein, hierzu die Lichtquelle derart auszuführen, dass die Messstrahlung einen die Eigenfluoreszenz eines zu bestimmenden Moleküls anregenden Spektralbereich aufweist, und den Lichtdetektor derart auszuführen, dass mindestens ein Spektralbereich der Nachweisstrahlung erfasst wird , in dem das Molekül Eigenfluoreszenzstrahlung emittiert.

Hierdurch wird eine Nachweismöglichkeit für Molekül-Mischungen, speziell mittelgroßer Moleküle wie Beta2-Microglobin, Albumin etc, geschaffen, deren Absorptionsbanden stark überlappt sind und die daher durch Absorptionsmessungen nicht hinreichend spezifisch nachgewiesen werden können. Der Einsatz zusätzlicher Marker wird überflüssig, so dass die praktische Durchführung sich vereinfacht, und es ist ein kontinuierlicher Nachweisprozess und damit die laufende ("Online") Überwachung von Betriebszuständen bzw. Prozessen möglich, bei denen der Anteil der erwähnten Moleküle in einer Flüssigkeit signifikant für den Prozessstatus ist.

In einer vorteilhaften Ausgestaltung ist vorgesehen, dass die Lichtquelle eine UV-Strahlungsquelle ist, die Tryptophan-Eigenfluoreszenz anregende Messstrahlung, insbesondere bei 280 nm, emittiert, und der Lichtdetektor eine Fotodiode oder ein Fotovervielfacher ist, die/der in einem Spektralbereich, in dem Tryptophan-Eigenfluoreszenzstrahlung emittiert wird, insbesondere oberhalb von 340 nm, empfindlich ist. Insbesondere ist dem Lichtdetektor ein schmalbandiger Filter vorgeschaltet, welches insbesondere einen Teilbereich des Spektralbereiches zwischen 340 nm und 400 nm als Nachweisbereich definiert.

Zur Erfassung der Eigenfluoreszenzstrahlung und ihrer Separierung von der (durch Absorptionseffekte veränderten) Messstrahlung ist vorteilhafter Weise vorgesehen, dass der Lichtdetektor eine Lichteinfallsrichtung hat, die gegenüber einer Emissionsrichtung der Lichtquelle geneigt ist.

Die Erfindung sieht vor, dass ein zusätzlicher Lichtdetektor zur Erfassung der Absorptionswirkung der Probenflüssigkeit bezogen auf den Probenflüssigkeits-Bestrahlungsabschnitt derart gegenüber der Lichtquelle angeordnet ist, dass seine Lichteinfallsrichtung mit der Emissionsrichtung der Lichtquelle zusammenfällt. Es wird hiermit also eine spektralphotometrische Auswertung von Absorptionseffekten mit der Auswertung der Eigenfluoreszenzstrahlung kombiniert, was zusätzliche Aussagemöglichkeiten bietet und gegebenenfalls eine präzisere Erfassung verschiedener Moleküle (etwa Proteinmoleküle, speziell Stoffwechsel-Abbauprodukte) in der zu untersuchenden Flüssigkeit ermöglicht.

In einer weiteren vorteilhaften Ausgestaltung ist die Nachweisvorrichtung für kontinuierlichen Betrieb eingerichtet und dann insbesondere zur OnlineÜberwachung von Dialysebehandlungen einsetzbar.

Bei der vorgeschlagenen Dialyseanordnung ist der Probenflüssigkeits-Bestrahlungsabschnitt als Abschnitt einer Messküvette oder Flüssigkeitsleitung ausgebildet, die von aus der Dialyseanordnung abgeführter, verbrauchter Dialyseflüssigkeit als Probenflüssigkeit durchströmt wird. In vorteilhafter Ausgestaltung einer solchen Anordnung ist eine mit der Auswertungsstufe der Nachweisanordnung verbundene Anzeigeeinheit zur Anzeige des Nachweisergebnisses oder hiervon abgeleiteter Daten und/oder eine Speichereinrichtung zur Speicherung des Nachweisergebnisses oder hiervon abgeleiteter Daten und/oder eine Übertragungseinrichtung zur Übertragung des Nachweisergebnisses oder hiervon abgeleiteter Daten zu einer externen Verarbeitungseinheit vorgesehen.

In ein er weiteren vorteilhaften Ausgestaltung ist die Auswertungsstufe der Nachweisvorrichtung mit einem Eingang einer Steuereinrichtung der Dialyseanordnung derart verbunden, dass der Betrieb der Dialyseanordnung in Abhängigkeit vom Erfassungsergebnis der Nachweisvorrichtung steuerbar ist.

Im Übrigen ergeben sich Vorteile und Zweckmäßigkeiten der Erfindung aus der nachfolgenden Beschreibung eines Ausführungsbeispiels anhand der Figuren. Von diesen zeigen:
- Fig. 1: eine schematische Darstellung einer Ausführungsform der Nachweisvorrichtung,
- Fig.2: eine schematische Darstellung einer Dialyseanordnung, in die eine Nachweisvorrichtung angebunden ist, und
- Fig. 3A und 3B: Darstellungen zur Erläuterung des Funktionsprinzips der Nachweisvorrichtung in einer Anwendung.

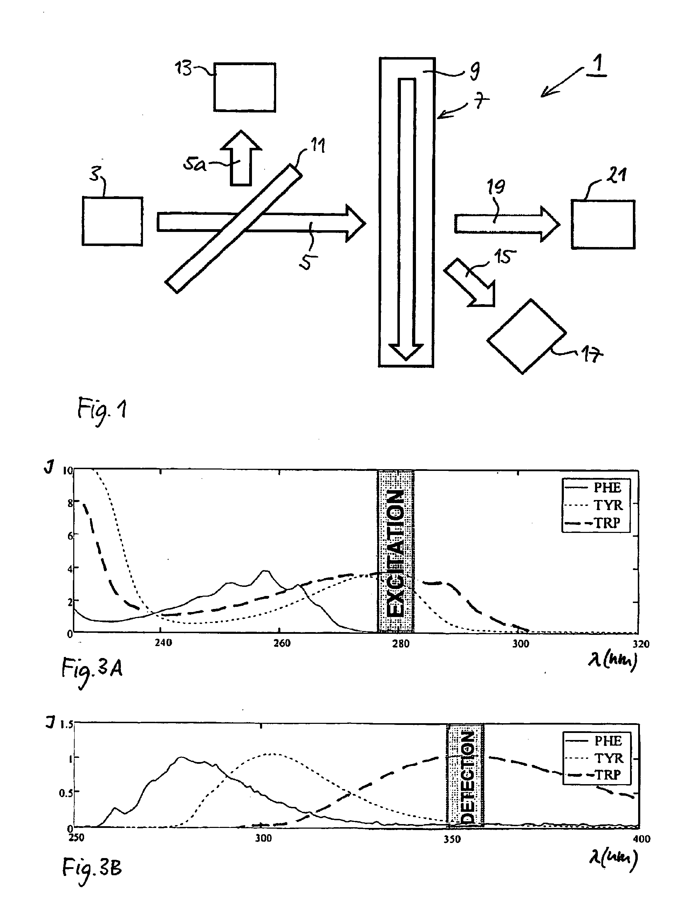

Fig. 1 zeigt skizzenartig eine Nachweisvorrichtung 1, bei der eine (an anderer Stelle auch als "Lichtquelle' bezeichnete) UV-LED 3 eine Messstrahlung 5 auf einen Probenflüssigkeits-Bestrahlungsabschnitt 7 aussendet, in dem sich eine Probenflüssigkeit 9 befindet. Durch einen halbdurchlässigen Spiegel 11 wird ein Teil 5a der Nachweisstrahlung zur Schaffung eines Referenzkanals zu einem ersten Detektor (nachfolgend auch als "Lichtdetektor" bezeichnet) 13 gelenkt. Die durch den halbdurchlässigen Spiegel 11 hindurchgehende Nachweisstrahlung 5 wirkt auf bestimmte in der Probenflüssigkeit 9 enthaltene Moleküle als Anregungsstrahlung zur Erzeugung einer Eigenfluoreszenzstrahlung 15. Diese gelangt zu einem zweiten Detektor 17, dessen Lichteinfallsrichtung gegenüber der Ausbreitungsrichtung der Nachweisstrahlung 5 geneigt ist und der somit keine durch den Probeflüssigkeits-Bestrahlungsabschnitt hindurchgehende Strahlung erfasst. Ein in Ausbreitungsrichtung der Nachweisstrahlung hindurchgehender Strahlungsanteil 19 wird jedoch durch einen dritten Detektor 21 erfasst. Mit diesem wird also eine Absorptionsmessung der Nachweisstrahlung ausgeführt. Einzelheiten der Auswertung der Fluoreszenz- sowie Absorptionssignale, speziell unter Heranziehung der im Referenzkanal gewonnenen Signale zur Kalibrierung, werden hier nicht beschrieben, weil sie dem Fachmann an sich geläufig sind.

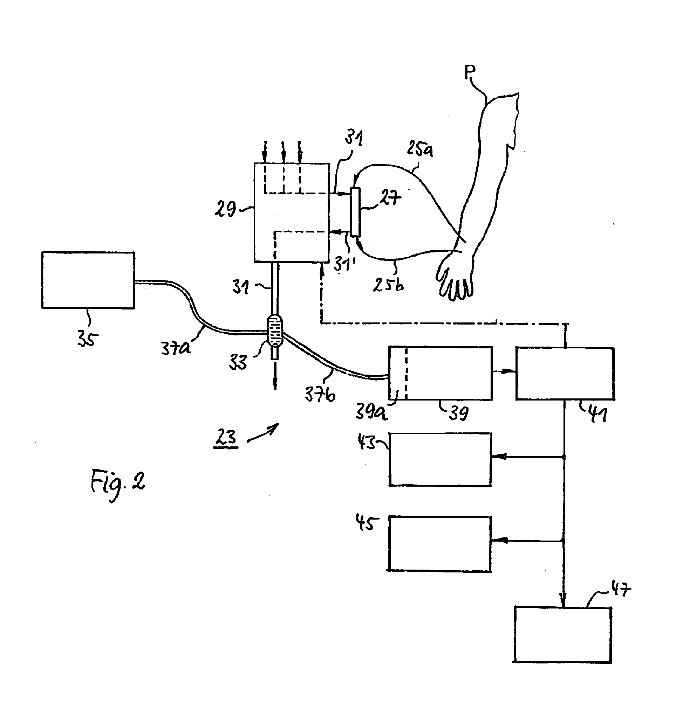

Fig. 2 zeigt in Art eines Blockschaltbildes eine Dialyseanordnung 23, bei der eine Nachweisvorrichtung zur Behandlungsüberwachung und -steuerung vorgesehen ist. Über Verbindungsschläuche 25a, 25b wird Blut eines Patienten P einer Dialysezelle 27 zugeführt bzw. aus dieser wieder in den Körper zurückgeführt. Aus mehreren (hier nicht näher spezifisierten) Komponenten wird in einem Dialysegerät 29 eine Dialyseflüssigkeit 31 hergestellt, die der Dialysezelle 27 zu- und aus dieser als verbrauchte Dialyseflüssigkeit 31' wieder abgeführt wird. Sie hat in der Dialysezelle 27 über eine dort vorgesehene Dialysemembran Abbauprodukte aus dem Körper des Patienten P aufgenommen, enthält also bestimmte Proteinmoleküle, deren Gehalt ein Maß für den Status der Dialysebehandlung ist.

Über eine Ausgangsleitung 31 des Dialysegeräts 29 wird die verbrauchte Dialyseflüssigkeit durch eine Messzelle 33 geleitet und nach Durchgang durch diese verworfen. Durch eine Strahlungsquelle 35 erzeugte (UV-)Messstrahlung gelangt über eine erste Glasfaserleitung 37a In die Messzelle, und über eine an der gegenüberliegenden Messzellen-Wandung geneigt zur Ankopplungs-Richtung der ersten Glasfaserleitung 37a angebrachte zweite Glasfaserleitung 37b gelangt Nachweisstrahlung (Eigenfluoreszenzstrahlung) zu einer Detektoreinrichtung 39. Diese enthält einen schmalbandigen Bandpass 39a, In einer abgewandelten Ausführung wird die Messstrahlung über ein optisches System, also ohne Glasfaserleitung, in die Messzelle eingekoppelt, und in gleicher Weise wird die Nachweisstrahlung aus der Messzelle ausgekoppelt.

Ausgangseitig ist mit der Detektoreinrichtung 39 eine Auswertungsstufe 41 zur quantitativen Auswertung des Detektorsignals verbunden. Die Auswertungsstufe 41 selbst ist ausgangsseitig einerseits mit einem Steuersignaleingang des Dialysegeräts 29 und andererseits mit einer Anzeigeeinheit 43, einer Speichereinheit 45 und einer Übertragungseinrichtung 47 verbunden, die zur Anzeige, Speicherung bzw. Übertragung des Nachweisergebnisses bzw. hiervon in der Auswertungsstufe 41 abgeleiteter Daten zu einer externen (zentralen) Verarbeitungseinheit ausgebildet sind.

Die Figuren 3A und 3B zeigen jeweils schematisch Spektraldiagramme zur Verdeutlichung der Funktionsweise einer Ausführung der Nachweisvorrichtung bei proteinhaltigen Probenflüssigkeiten, die Phenylalanin PHE, Tyrosin TYR und Tryptophan TRP enthalten. Fig. 3A zeigt die Anregungswellenlänge dieser Moleküle, wobei ein zweckmäßiger Anregungs-Spektralbereich EXCITATION markiert ist, und Fig. 3B zeigt die Wellenlängen der Eigenfluoreszenzstrahlung der drei Moleküle. Hier ist ein zweckmäßiger Nachweisbereich DETECTION für das Tryptophan-Molekül markiert. Es ist zu erkennen, dass die Anregungs-Wellenlängen um 280 nm liegen, während der hier ausgewählte Detektions-Spektralbereich bei 350 nm und etwas oberhalb liegt. Hier hat das Tryptophan-Molekül ein Maximum der Eigenfluoreszenz, während die Eigenfluoreszenzspektren von Phenylalanin und Tyrosin in diesem Spektralbereich keine Nennenswerte Intensität haben. Eine entsprechende Detektor- bzw. Filtereinstellung ermöglicht also einen hochselektiven Nachweis des Tryptophan.

Die Ausführung der Erfindung ist nicht auf die hier beschriebenen Beispiele und hervorgehobene Aspekte beschränkt, sondern ebenso in zahlreichen Abwandlungen möglich, die im Rahmen fachgemäßen Handelns liegen. Insbesondere sind andere Lichtquellen- und Detektorausführungen möglich und in Abhängigkeit von den konkreten Anregungs- und Emissionswellenlängen zu detektierender Moleküle sinnvoll zu wählen.

## Patentansprüche

1. Dialyseanordnung (23) mit einer Nachweisvorrichtung (1) zum quantitativen Nachweis vorbestimmter Moleküle, insbesondere von mittelgroßen Protein-Molekülen, in einer Probenflüssigkeit, mit einer Lichtquelle (3) zur Aussendung von Messstrahlung, einem Probenflüssigkeits-Bestrahlungsabschnitt (7) und einem Lichtdetektor (17) zur Erfassung von den Probenflüssigkeits-Bestrahlungsabschnitt verlassender Nachweisstrahlung sowie einer dem Lichtdetektor (17) nachgeordneten Auswertungsstufe (41) zur Auswertung des Detektorsignals, wobei die Lichtquelle (3) derart ausgeführt ist, dass die Messstrahlung einen die Eigenfluoreszenz eines zu bestimmenden Moleküls anregenden Spektralbereich aufweist, und der Lichtdetektor (17) derart ausgeführt ist, dass mindestens ein Spektralbereich der Nachweisstrahlung erfasst wird, in dem das Molekül Eigenfluoreszenzstrahlung emittiert, wobei ein zusätzlicher Lichtdetektor (21) zur Erfassung der Absorptionswirkung der Probenflüssigkeit bezogen auf den Probenflüssigkeits-Bestrahlungsabschnitt derart gegenüber der Lichtquelle angeordnet ist, dass seine Lichteinfallsrichtung mit der Emissionsrichtung der Lichtquelle (3) zusammenfällt, wobei der Probenflüssigkeits-Bestrahlungsabschnitt (7) als Abschnitt einer Messküvette (33) oder Flüssigkeitsleitung ausgebildet ist, die von aus der Dialyseanordnung (23) abgeführter, verbrauchter Dialyseflüssigkeit als Probenflüssigkeit durchströmt wird.

2. Dialyseanordnung nach Anspruch 1, wobei die Lichtquelle (3) eine UV-Strahlungsquelle ist, die Tryptophan-Eigenfluoreszenz anregende Messstrahlung, insbesondere bei 280 nm emittiert, und der Lichtdetektor (17) eine Fotodiode oder ein Fotovervielfacher ist, die/der in einem Spektralbereich, in dem Tryptophan-Eigenfluoreszenzstrahlung emittiert wird, insbesondere oberhalb von 340 nm, empfindlich ist.

3. Dialyseanordnung nach Anspruch 1 oder 2, wobei dem Lichtdetektor (17) ein schmalbandiges Filter vorgeschaltet ist, welches insbesondere einen Teilbereich des Spektralbereiches zwischen 340 nm und 400 nm als Nachweisbereich definiert.

4. Dialyseanordnung nach einem der vorangehenden Ansprüche, wobei der Lichtdetektor (17) eine Lichteinfallsrichtung hat, die gegenüber einer Emissionsrichtung der Lichtquelle (3) geneigt ist.

5. Dialyseanordnung nach einem der vorangehenden Ansprüche, wobei die Nachweisvorrichtung (1) für kontinuierlichen Betrieb ausgeführt ist.

6. Dialyseanordnung nach Anspruch 1, wobei eine mit der Auswertungsstufe (41) der Nachweisanordnung (1) verbundene Anzeigeeinheit (43) zur Anzeige des Nachweisergebnisses oder hiervon abgeleiteter Daten und/oder eine Speichereinrichtung (45) zur Speicherung des Nachweisergebnisses oder hiervon abgeleiteter Daten und/oder eine Übertragungseinrichtung (47) zur Übertragung des Nachweisergebnisses oder hiervon abgeleiteter Daten zu einer externen Verarbeitungseinheit vorgesehen ist.

7. Dialyseanordnung nach Anspruch 1 oder 6, wobei die Auswertungsstufe (41) der Nachweisvorrichtung mit einem Eingang einer Steuereinrichtung der Dialyseanordnung (29) derart verbunden ist, dass der Betrieb der Dialyseanordnung in Abhängigkeit vom Erfassungsergebnis der Nachweisvorrichtung steuerbar ist.

## Claims

1. Dialysis apparatus (23) having a detection device (1) for quantitative detection of predetermined molecules, in particular of medium-sized protein molecules in a sample liquid, having a light source (3) for emitting measurement radiation, a section (7) for irradiating the sample liquid and a light detector (17) for measuring the detection radiation exiting the section for irradiating the sample liquid, as well as an analysis stage (41) located downstream of the light detector (17) for analyzing the detector signal, wherein the light source (3) is designed such that the measurement radiation has a spectral range exciting the autofluorescence of a molecule to be determined, and the light detector (17) is designed such that at least one spectral range of the detection radiation is measured, in which the molecule emits autofluorescence radiation, wherein an additional light detector (21) for measuring the effect of the absorption of the sample liquid with regard to the section (7) for irradiating the sample liquid is arranged opposite to the light source such that its direction of incidence of light coincides with the direction of emission of the light source (3),
wherein the section (7) for irradiating the sample liquid is designed in the form of a section of a measurement cell (33) or a liquid conduit, which is perfused by spent dialysis fluid discharged from the dialysis apparatus (23) as sample liquid.

2. Dialysis apparatus according to claim 1, wherein the light source (3) is a UV-radiation source emitting measurement radiation exciting the intrinsic fluorescence of tryptophan, in particular at 280 nm, and the light detector (17) is a photodiode or a photomultiplier, which is sensitive in a spectral range, in which the intrinsic fluorescence radiation of tryptophan is emitted, in particular above 340 nm.

3. Dialysis apparatus according to claim 1 or 2, wherein a narrowband filter, which particularly defines a part of the spectral range between 340 nm and 400 nm as detection range, is connected upstream of the light detector (17).

4. Dialysis apparatus according to one of the preceding claims, wherein the light detector (17) has a direction of incidence of light which is inclined with respect to a direction of emission of the light source (3).

5. Dialysis apparatus according to one of the preceding claims, wherein the detection device (1) is designed for continuous operation.

6. Dialysis apparatus according to claim 1, wherein a display unit (43) connected with the analysis stage (41) of the detection device (1) for displaying the analysis result or data derived there from and/or a storage device (45) for storage of the analysis result or data derived there from and/or a transmission device (47) for transmission of the analysis result or data derived there from to an external processing unit is provided.

7. Dialysis apparatus according to claim 1 or 6, wherein the analysis stage (41) of the detection device is connected with an input of a control device of the dialysis apparatus (29) such that the operation of the dialysis apparatus is controllable in dependence on the detection result of the detection device.

## Revendications

1. Appareil de dialyse (23) ayant un dispositif de détection (1) pour la détection quantitative de molécules prédéterminées dans un échantillon de liquide, et en particulier de molécules de protéine de taille moyenne, ayant une source de lumière (3) pour émettre un rayonnement de mesure, une section (7) pour irradier l'échantillon de liquide et un détecteur de lumière (17) pour mesurer le rayonnement de détection quittant la section pour irradier l'échantillon de liquide, ainsi qu'une étape d'analyse (41) localisée en aval du détecteur de lumière (17) pour analyser le signal de détection, dans lequel la source de lumière (3) est conçue de telle façon que le rayonnement de mesure a un domaine spectral qui excite l'auto-fluorescence d'une molécule à être déterminée, et le détecteur de lumière (17) est conçu de telle façon qu'au moins un domaine spectral du rayonnement de détection est mesuré, dans lequel la molécule émette le rayonnement d'auto-fluorescence, dans lequel un détecteur de lumière supplémentaire (21) pour mesurer l'effet d'absorption de l'échantillon de liquide par référence à la section (7) pour irradier l'échantillon de liquide est disposé du coté opposé de la source de lumière de telle façon que sa direction d'incidence de la lumière coïncide avec la direction d'émission de la source de lumière (3), dans lequel la section (7) pour irradier l'échantillon de liquide est conçue dans la forme d'une section d'une cellule de mesure (33) ou d'un conduit de liquide qui est traversée par le liquide de dialyse consommé sortant de l'appareil de dialyse (23) comme l'échantillon de liquide.

2. L'appareil de dialyse selon la revendication 1, dans lequel la source de lumière (3) est une source de rayonnement UV qui émette un rayonnement de mesure excitant la fluorescence intrinsèque du tryptophane, en particulier à 280 nm, et le détecteur de lumière (17) est une photodiode ou un photomultiplicateur, qui est sensible dans un domaine spectral dans lequel la fluorescence intrinsèque du tryptophane est émise, particulièrement au dessus de 340 nm.

3. L'appareil de dialyse selon la revendication 1 ou 2, dans lequel un filtre à bande étroite qui définit en particulier une partie du domaine spectral comprise entre 340 nm er 400 nm comme domaine de détection, est connecté en amont du détecteur de lumière (17).

4. L'appareil de dialyse selon une des revendications précédentes, dans lequel le détecteur de lumière (17) a une direction d'incidence de la lumière qui est inclinée par rapport à une direction d'émission de la source de lumière (3).

5. L'appareil de dialyse selon une des revendications précédentes, dans lequel le dispositif de détection (1) est conçu pour un fonctionnement continu.

6. L'appareil de dialyse selon la revendication 1, dans lequel une unité d'affichage (43) connectée à l'étape d'analyse (41) du dispositif de détection (1) pour afficher le résultat d'analyse ou les données issues là par et/ou un dispositif de sauvegarde (45) pour sauvegarder le résultat d'analyse ou les données issues là par et/ou un dispositif de transmission (47) pour la transmission du résultat d'analyse ou des données issues là par une unité externe de traitement est fourni(e).

7. L'appareil de dialyse selon la revendication 1 ou 6, dans lequel l'étape d'analyse (41) du dispositif de détection est connectée à une entrée du dispositif de contrôle de l'appareil à dialyse (29) de telle façon que le fonctionnement de l'appareil de dialyse est contrôlable en dépendance du résultat de détection du dispositif de détection.
